# EUROPEAN PATENT SPECIFICATION

(11) **EP 1 981 457 B1**
(45) Date of publication and mention of the grant of the patent: **25.03.2015**
(21) Application number: 07763626.4
(22) Date of filing: 06.02.2007
(51) Int. Cl.: A61F 13/00, C03C 12/00

(54) **SURGICAL WOUND DRESSING**
CHIRURGISCHE WUNDAUFLAGE
PANSEMENT CHIRURGICAL

(30) Priority: 07.02.2006 US 765921 P
(43) Date of publication of application: 22.10.2008
(73) Proprietor: Smith & Nephew, Inc., Memphis, TN 38116 (US)
(72) Inventor: MULLIGAN, Sharon, Bristol, RI 02809 (US)
(74) Representative: Gray, James
(86) International application number: PCT/US2007/003079
(87) International publication number: WO 2007/092405

(56) References cited:
- WO-A1-2006/048246
- US-A- 4 664 662
- US-A1- 2004 054 338
- US-A1- 2005 137 539
- US-B2- 6 979 324

## Description

### CROSS-REFERENCE TO RELATED APPLICATIONS

### BACKGROUND

### 1. Technical Field

The present disclosure relates to an apparatus for treating an open wound, and, more specifically, relates to a wound dressing system for use in negative pressure therapy.

### 2. Description of Related Art

Wound closure involves the migration of epithelial and subcutaneous tissue adjacent the wound towards the center of the wound until the wound closes. Unfortunately, closure is difficult with large wounds or wounds that have become infected. In such wounds, a zone of stasis (i.e. an area in which localized swelling of tissue restricts the flow of blood to the tissues) forms near the surface of the wound. Without sufficient blood flow, the epithelial and subcutaneous tissues surrounding the wound not only receive diminished oxygen and nutrients, but, are also less able to successfully fight microbial infection and, thus, are less able to close the wound naturally. Such wounds have presented difficulties to medical personnel for many years.

Wound dressings have been used in the medical industry to protect and/or facilitate healing of open wounds. One technique has been to use negative pressure therapy, which is also known as suction or vacuum therapy. A variety of negative pressure devices have been developed to allow excess wound fluids, i.e., exudates to be removed while at the same time isolating the wound to protect the wound and, consequently, effect recovery time. Various wound dressings have been modified to promote the healing of open wounds.

Issues that continually need to be addressed when using a wound dressing in negative pressure therapy include ease of use, efficiency of healing a wound, and sufficient drainage of wound exudates. Thus, there remains a need to constantly improve negative pressure wound dressings for open wounds.

### SUMMARY

Accordingly, the present disclosure is directed to a wound dressing system including a fluid permeable support member for positioning within a wound and adapted to generally conform to a topography of a wound, a plurality of beads supported by the support member, an outer member for positioning over the wound to substantially enclose the beads and a conduit for supplying reduced pressure to the wound, characterized by some of the plurality of beads each having a bore therethrough.

The support member is adapted to permit exudates from the wound to pass therethrough. The beads are characterized by having sufficient rigidity to substantially maintain respective shapes thereof to thereby facilitate passage of the exudates through spaces or passages defined between adjacent beads. The beads may comprise glass, an acrylic or a polymeric material. At least some of the beads may comprise an outer layer of medicament. Alternatively, at least some of the beads may include a bore therethrough to permit passage of exudates. Medicaments may be disposed with the bores of these beads. In one embodiment, the beads are connected along an elongated or string member. In another embodiment, the beads are bonded or mounted to the support member. The beads may be absorbable or non absorbable.
If fabricated from an absorbable material, the rate of absorption of the beads may be controlled, preselected, etc. to maintain the integrity of the beads during a sufficient period of time to provide channels through the bead arrangement to facilitate drainage of the exudates. The beads may further be capable of absorbing some of the exudates. The beads may be periodically removed from the wound area and replaced with new beads. Alternatively, the beads may be adapted to remain during the entire healing process.

The support member may comprise a polymeric or fabric material. The support member may be an enclosure member or pouch which houses the beads. Multiple pouches are also envisioned. The outer member is adapted to form a substantially liquid-tight seal about the wound. The outer member may comprise a sem i-permeable material.

A negative pressure source is in fluid communication with the conduit. The negative pressure source includes a vacuum pump.

In another embodiment, the wound dressing system includes a fluid permeable enclosure pouch for positioning within a wound and adapted to permit exudates from the wound to pass therethrough, a plurality of beads disposed within the enclosure pouch, an outer member adapted for positioning over the wound to substantially enclose the beads and negative pressure means for supplying reduced pressure to the wound. The beads have sufficient rigidity to substantially maintain respective shapes thereof to thereby facilitate passage of the exudates through spaces defined between adjacent beads.

In another preferred embodiment, the wound dressing system includes a frame dimensioned for positioning within a wound bed and having a series of interconnected walls defining spaced cells, an outer member adapted for positioning over the wound and adapted to form a liquid tight seal about the wound and a conduit for supplying reduced pressure to the wound bed. The cells have sufficient rigidity to substantially maintain their original configurations in the presence of reduced pressure and have openings dimensioned to permit passage of wound exudates therethrough. At least one of the cells define a substantially polygonal shape, e.g., triangular, square or hexagonal or may be arcuate. The frame preferably comprises silicon.

### BRIEF DESCRIPTION OF THE DRAWINGS

Various embodiments of the wound dressing system of the present disclosure are described herein with reference to the drawings wherein:
FIG. 1 is a side view of the wound dressing system of the present disclosure illustrating the wound dressing and negative pressure source;
FIG. 2 is a side cross-sectional view of one embodiment of a bead for use with the wound dressing of the system of FIG. 1;
FIG. 3 is a side cross-sectional view of another embodiment of a bead for use with the wound dressing of the system of FIG. 1;
FIG. 4 is a graphical representation of negative or pump set pressure as a function of time for the wound dressing of the system of FIG. 1 and a prior art dressing with foam;
FIGS. 5A and 5B are graphical representations of negative pressure levels taken at any point through the thickness of the dressing for the wound dressing of the system of FIG. 1 and prior art gauze and foam dressings;
FIG. 6 is a graphical representation of the pressure profile at a wound site for a dry wound for the wound dressing of the system of FIG. 1 and a prior art foam dressing;
FIG. 7 is a graphical representation of the pressure profile at wound site for a wet wound for the wound dressing of the system of FIG. 1 and a prior art foam dressing;
FIG. 8A and 8B are graphical representations of vacuum levels for a tunneling wound dressing of the system of FIG. 1 and a prior art foam dressing;
FIG. 9 is a graphical illustration illustrating vacuum levels vs. time for large and small beads used with the wound dressing of the system of FIG. 1;
FIG. 10 is a side view of an alternative embodiment of the wound dressing of the system of FIG. 1, incorporating a support member in the form of an enclosure member for accommodating the beads;
FIG. 11 is a side view of another alternative embodiment of the wound dressing of the system of FIG. 1, illustrating a plurality of enclosure members for accommodating the beads;
FIG. 12 is a view of an alternate embodiment of the system of FIG. 1, illustrating the beads of the wound dressing attached along an interconnecting member;
FIG. 13 is a view of another embodiment of the system of FIG. 1 illustrated positioned within the wound bed;
FIGS. 14 and 15 are top and side views respectively of the support sheet of the system of FIG. 13 illustrating the beads mounted to the support sheet; and
FIG. 16 is a view of yet another embodiment of the system of FIG. 1 incorporating a lattice frame matrix within the wound dressing.

### DESCRIPTION OF THE PREFERRED EMBODIMENTS

The wound dressing system of the present disclosure promotes healing of a wound in conjunction with negative pressure therapy. One preferred wound dressing of the system includes a plurality of beads supported by a support layer. The beads conform to the shape of the wound while allowing the air and exudates to flow through the dressing, thereby promoting a moist environment and facilitating healing of the wound.

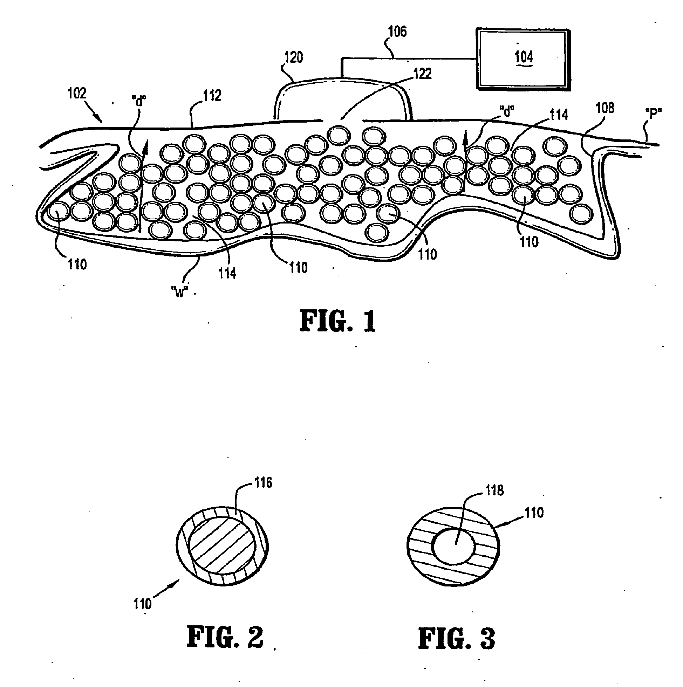

Referring now to FIG. 1, one preferred embodiment of the wound dressing system 100 of the present disclosure is illustrated. Wound dressing system 100 includes wound dressing 102 and negative pressure means or source 104 in fluid communication with the wound dressing 102 through conduit, identified schematically as reference numeral 106. Wound dressing 102 includes several components, namely, support layer or member 108, a plurality of beads 110 supported by the support member 108 and outer layer or cover member 112.

Support member 108 is adapted to substantially conform to the topography of a wound bed "w". Support member 108 is substantially porous to permit exudates to pass from the wound bed "w" through the support member 108. "Porous" as used herein refers to a material which contains numerous small perforations or pores which allow wound fluids of all kinds to pass through the material. Support member 108 may also be non-adherent. "Non-adherent" as used herein refers to a material that does not adhere to tissues in and around the wound bed. This configuration allows fluid and exudates to flow uninhibited through support member 108 with minimal "sticking" of support member 108 to the wound bed "w".

The passage of wound exudates through the support member 108 is preferably unidirectional such that wound exudates do not flow back to the wound bed "w". This unidirectional flow feature could be in the form of directional apertures imparted into the material layer, a lamination of materials of different absorption to the support member 108 or specific material selection that encourages directional flow. However, a bidirectional layer for the purposes of supplying medicine or anti-infectives to the wound bed "w" is also envisioned and will be described hereinafter.

In addition, agents such as hydrogels and medicaments could be bonded or coated to the support member 108 to reduce bioburden in the wound, promote healing and reduce pain associated with dressing changes or removal. Medicaments include, for example, antimicrobial agents, growth factors, antibiotics, analgesics, debridement agents, and the like. Furthermore, when an analgesic is used, the analgesic could include a mechanism that would allow the release of that agent prior to dressing removal or change.

Support member 108 may be constructed from a variety of different materials. These may include but are not limited to synthetic non absorbable polymer fibers such as carbonized polymers, polyethylene (PET), polypropylene (PP), polytetrafluoroethylene (PTFE), Nylon, arimids, Kevlar, polyethylene terephthalate (PET) or natural fibers such as cellulose. It is envisioned that support member 108 may be transparent to allow improved visual capacity and a better view of wound bed "w". Moreover, support member 108 may be constructed of a fabric which could be woven, nonwoven (including meltblown), knitted or composite structures such as spun bonded fabrics. Exemplary materials used as support member 108 are sold under the trademark EXCILON^{™} by Kendall Corp, a division of TycoHealthcare.

With reference again to FIG.1, beads 110 will now be discussed. Beads 110 are preferably substantially rigid so as to maintain their shapes for at least a predetermined period of time during healing. In this regard, beads 110 when arranged within the wound bed "w" define spaces or passages 114 therebetween to permit wound exudate to pass through the passages 114. The sizes of beads 110 can vary, but they should be sized to achieve the proper pore size through the bead arrangement to facilitate cell proliferation and allow fluid and air to be evacuated from the wound. A porosity in the range of 10-1000µm has been found beneficial in stimulating cell proliferation and in allowing fluid and air to be evacuated from the wound. Beads 110 work in conjunction with support member 108 to conform to the wound bed "w" while allowing drainage of wound exudates and release of air from the wound bed "w" without clogging. As the negative pressure is applied, beads 110 will move and readjust their respective positions to prevent painful ingrowth that can occur with current foam designs.

Beads 110 are manufactured from a suitable biocompatible material. Beads 110 may be antimicrobial beads, beads with growth factors, medicaments, antibiotics, analgesics, and healing factors such as vitamins, nutrients and the like. These beads 110 are preferably non-adherent and may be bioabsorbable over a predetermined period of time. Acrylic (PMMA) can be used for its clarity and would also provide the ability for the clinician to see the wound without removing the dressing. Other materials that could be used are polycarbonate, polystyrene, PVC, ABS, SAN, glass or silicone. Bioabsorbable polymers could also be used, e.g., polylactide (PLA), polyglycolide (PGA), Chitosan, polyethylene oxide (PEO) or polyethylene glycol (PEG).

Beads 110 are preferably substantially rigid for at least a predetermined period of time during healing so as to maintain the desired spacing to thereby define passages 114 through the bed of beads for passing of wound exudates, air, etc. Thus, beads 110 may be nonabsorbable. Alternatively, beads 110 and bead size may be partially or fully absorbable. With an absorbable material, the rate of absorption of beads 110 is selected to maintain the desired rigidity during a predetermined period of healing. One skilled in the art may select the materials of fabrication of beads 110 to reach these objectives. The dissolution rate of beads 110 is dependent on material selection, bead size (surface area of bead in contact with fluids), amount of fluid in wound bed and the temperature. Some or all of beads 110 could be designed to remain rigid for the entire time that the dressing will remain in place on the patient, 1 day to 1 week. This will maintain air and fluid flow from the wound.Some of beads 110 could be designed to dissolve over this time period, 1 day to 1 week, to release any active ingredients incorporated into the beads 110. Additional dissolution of beads 110 could be timed to coincide with planned dressing changes to prevent tissue from growing into the beads and causing trauma upon removal of dressing.

Multiple variations of beads 110 are also contemplated. For example, beads 110 may further include a medicinal layer 116 configured to distribute medicaments into the wound bed "w" as depicted in FIG. 2. Medicinal layer 116 may contain any of the medicaments discussed hereinabove in connection with the discussion of support member 108. These medicaments are preferably released into the wound bed "w" at a desired rate to optimize healing and/or reduce pain. Alternatively, as depicted in FIG. 3, beads 110 may be porous including a central bore 118 thereby providing an additional passage for the wound exudate and/or circulation of air about the wound site. It is also envisioned that these bores 118 may contain bioabsorbable materials such as antimicrobials, anti-infectives, or other medicaments. Once the medicaments are absorbed into the wound bed "w", the bores 118 remain, and flow of air and wound exudates is facilitated.

Referring again to FIG. 1, outer member 112 encompasses the perimeter of the wound dressing 100 to surround wound bed "w" and to provide a seal around the perimeter "p" of the wound bed "w". For instance, the sealing mechanism may be any adhesive bonded to a layer that surrounds the wound bed "w". The adhesive must provide acceptable adhesion to the tissue surrounding the wound bed "w", e.g., the periwound area, and be acceptable for use on the skin without contact deteriorization (e.g., the adhesive should preferably be non-irritating and non-sensitizing). The adhesive may be permeable to permit the contacted skin to breathe and transmit moisture. Additionally, the adhesive could be activated or de-activated by an external stimulus such as heat or a given fluid solution or chemical reaction. Adhesives include, for example, medical grade acrylics like the adhesive used with CURAFOAM ISLAND™ dressing of TycoHealthcare Group, LP or any silicone or rubber based medical adhesives that are skin friendly and non irritating. Outer member 112 may be provided with an adhesive backing and/or alternatively, an adhesive may be applied to the outer member 112 and/or skin during the procedure.

Outer member 112 is typically a flexible material, e.g., resilient or elastomeric, that seals the top of wound dressing 100. Exemplary flexible materials include the transparent dressing manufactured under the trademark Polyskin II by Kendall Corp, a division of Tyco Healthcare Group LP. Preferably, outer member 112 is transparent and provides a barrier to microbes and fluid containment. Outer member 112 may be manufactured from a permeable plastic film providing it with a high moisture vapor transmission rate (MVTR) to allow the passage of exudates through the film. Such films could be manufactured from polyurethanes, breathable polyolefins, or copolyesters. The transparency of outer member 112 permits a visual review of the status of the wound dressing and more particularly, the wound itself. Alternatively, outer member 112 may be impermeable to moisture vapors.

Outer member 112 may include an access door (not shown) to provide access to the interior of the wound dressing 102 and/or the wound bed "w". The door could be a flap integrally formed with outer member 112 or a separate component connected to outer member 112 via a hinge or the like. The door may be resealable to maintain the integrity of the wound dressing 102 and to provide an airtight seal relative to the outer member 112. The door preferably provides access to the wound bed "w" to enable the clinician to monitor the status of the wound, adjust, add or remove, beads 110, or apply additional medical treatment to the wound such as growth factors, debriders, or other wound healing agents as needed.

With reference again to FIG.1, negative pressure source 104 applies vacuum through conduit 106 to the wound. In one embodiment, a manual pump is utilized as the negative pressure source. One suitable manual pump is disclosed in commonly assigned U.S. Patent No. 5,549,584 to Gross, the entire contents of which are hereby incorporated herein by reference. Alternatively, an automated pump may be utilized. Typically, the negative pressure generated within the wound "w" by negative pressure source 104 is in a range between about 20 mmHg to about 500mmHg, more preferably, about 75 mmHg and about 125 mmHg. The automated pump may be in the form of wall suction such as those available in an acute or sub-acute care facility. The automated pump may be in the form of a portable pump. The portable pump may be in the form of a small or miniature pump that maintains or draws adequate and therapeutic vacuum levels. In a preferred embodiment, the pump is a portable, lightweight, battery operated, suction pump which attaches to the proximal end of the tubing. Typically, negative pressure source 104 has regulation means to apply the optimal vacuum pressure for healing the wound. Furthermore, the negative pressure source 104 would preferably contain a mechanism to detect a leak in the system if the optimal vacuum pressure is not achieved. Preferably, negative pressure source 104 would also contain an indicator (not shown) or transducer to detect the pressure within the wound or wound dressing. A peristaltic pump may be used as negative pressure 104. Typical peristaltic pumps include a plurality of rollers that are driven along in a circular path by an electric motor (or other suitable prime mover) in a rotational direction. As is common in most such peristaltic pumps, rollers periodically contact and compress the tubing and as the rollers move along the circular path, successive pockets of air are suctioned through the tubing for suctioning exudates from the wound. Preferably, the peristaltic pump delivers about 125 mmHg to the wound bed at 50cc per minute.

In a preferred embodiment, conduit 106 is in the form of flexible tubing and is in fluid communication with vacuum connector 120. Vacuum connector 120 is mounted to wound dressing 102. Vacuum connector 120 may be secured to the outer surface of outer member 112 via adhesives or the like, or alternatively, may be at least partially introduced within opening 122 in outer member 112 and secured within the opening 122. Vacuum connector 120 may be applied to wound dressing 102 at the operative site or may be pre-connected to the wound dressing 102. In the alternative, wound dressing 102 may be devoid of vacuum connector 122 whereby conduit 106 would directly access the interior of wound dressing 102.

In use, support member 108 is placed within the wound bed "w" as shown in FIG. 1. Beads 110 are positioned within support member 108. Beads 110 supported within support member 108 will arrange themselves to conform to the shape of the wound bed "w". In particular, beads 110 will migrate into remote areas of the wounds, i.e., "tunnel" into the wound as shown in FIG. 1. Outer member 112 is then placed in contact with skin "s" and may be secured about its periphery to the wound "w" with adhesives or the like. Vacuum connector 120 is then secured to outer member 112 adjacent opening 122 of the outer member 122. Conduit 106 is then connected to vacuum connector 120. Negative pressure source 104 is then activated creating a reduced pressure state within wound dressing 102. As the pumping progresses, beads 110 maintain their shape thereby creating and/or maintaining passageways 114 for the wound exudates to flow. Wound exudates flow through support member 108 through beads 110 (as depicted by directional arrows "d") and out conduit 106. Preferably, the wound exudates are deposited within a canister or container in line with conduit 106 and negative pressure source 104.

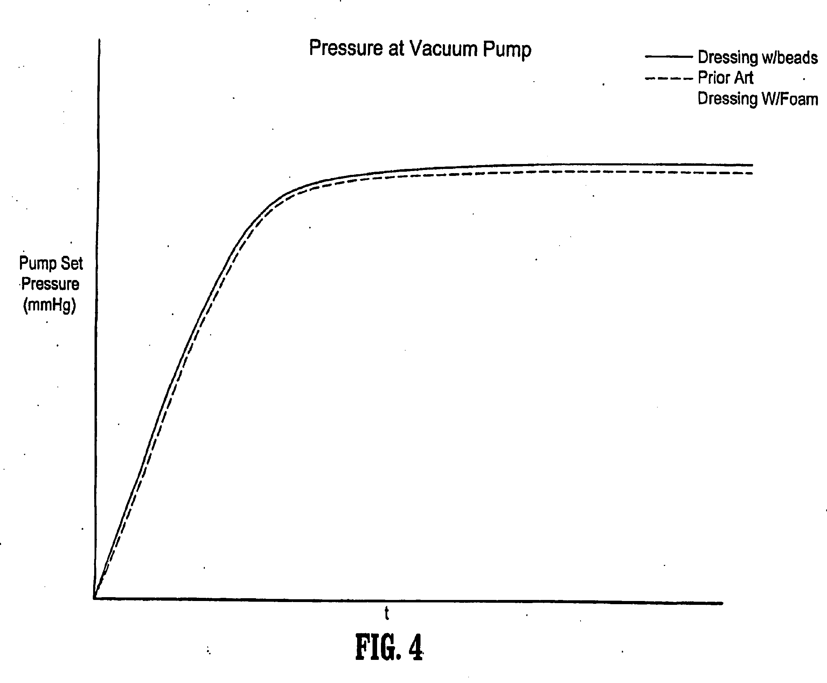

When a typical vacuum pump (e.g., negative pressure source 104) is activated and set at a specific set point, the pump will begin to draw pressure until it achieves the set point. The vacuum reading at the pump will stay at this level until the set point is changed, the pump is turned off, or there is a major leak in the system that overcomes the pumps ability to continue to achieve this level. FIG. 4 illustrates vacuum pressure versus time (t) as measured at the vacuum pump for a negative pressure wound therapy pump as the pump is turned on and pumps down to its set point. The pump pressure reads the same for any dressing that is used in the wound (e.g., either wound dressing 102 with beads 110 or a prior art wound dressing incorporating a foam layer) because the pressure sensor controlling the pump is next to the pump. In this example, using either the dressing of the present disclosure or a foam dressing of the prior art will result in the same steady state pressure reading at the pump.

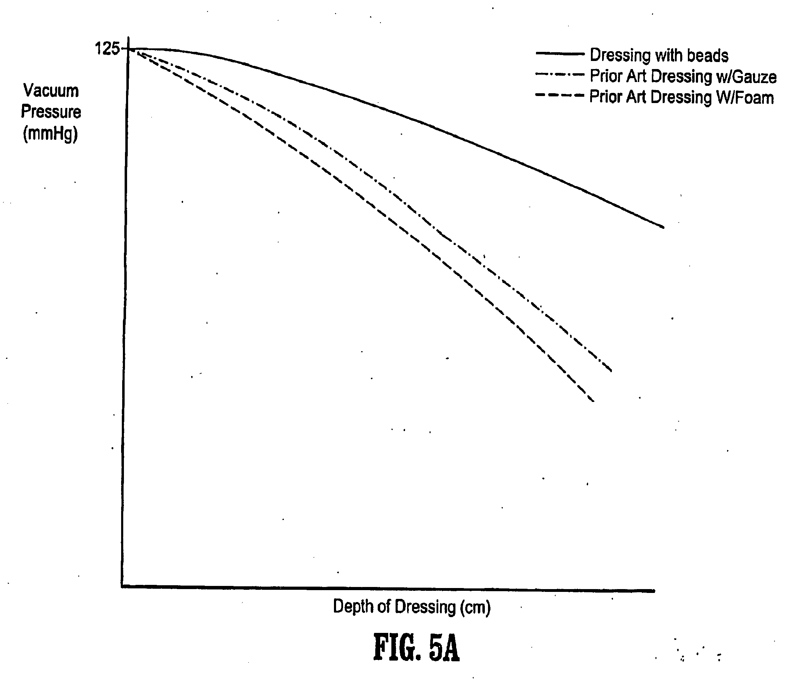

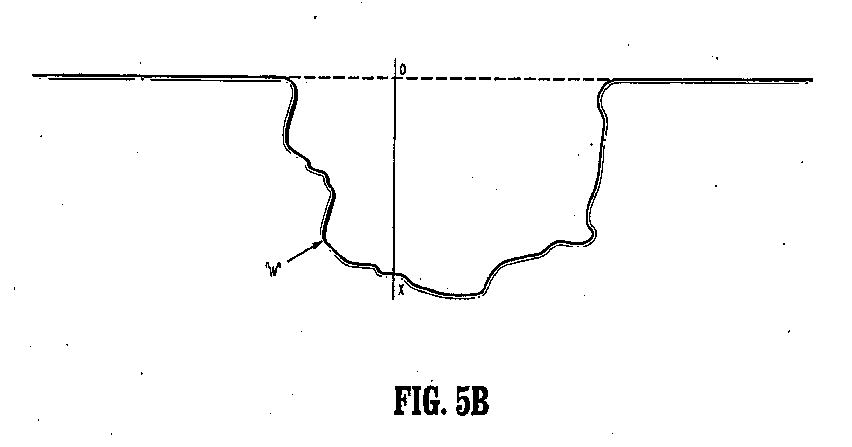

Although the pressure readings at the pump may be the same for different dressings, the actual pressure at the wound site will be different depending on the dressing utilized. A difference in actual vacuum level at the wound site may be seen depending on the dressing used due to the differences in the ability of dressings to evacuate effectively. FIGS. 5A and 5B show the relationship between the depth of the dressing (in cm) and actual pressure level at any point through the thickness of the dressing from the top surface (0) to the wound surface (x) for different dressing materials. Because a conventional foam or gauze dressing collapses under vacuum thus restricting air flow from the wound site, the pressure at the wounds site never actually reaches the set point on the pump. As can be seen, wound dressing 102 with beads 110 of the present disclosure maintains the higher level of vacuum even at thicker dressing depth (compared to conventional foam and gauze filled dressings) because the beads 110 are not substantially collapsible and an open air pathway is maintained through the interstices.

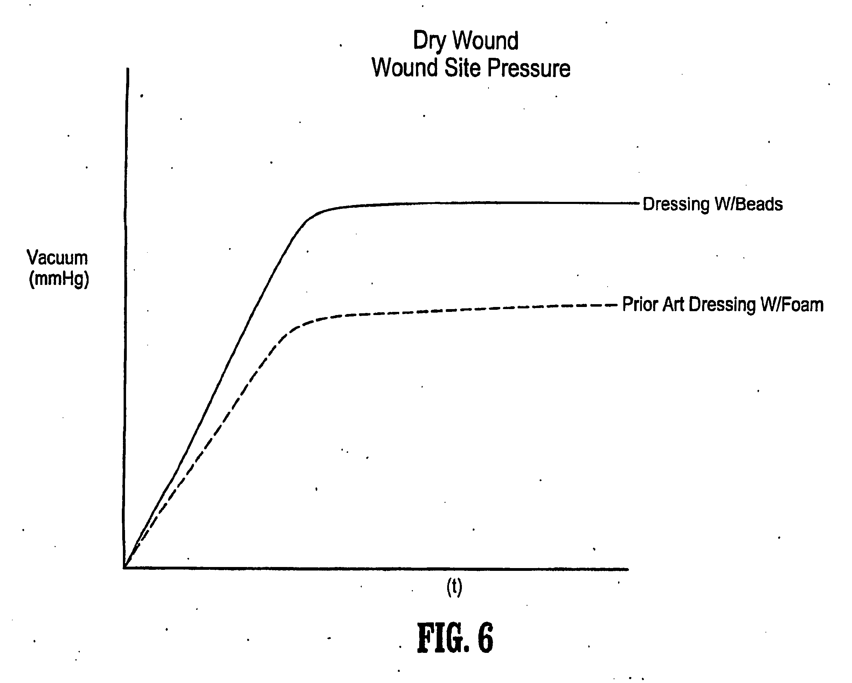

The difference in flow restriction between dressing materials can also affect the pump down time and the achieved vacuum level at the wound site. FIG. 6 shows the pressure profile at the wound site for a dry wound, i.e., no influence of wound exudate, for the dressing 102 of the present disclosure and the common dressing material of foam. The dressing of the present disclosure shows an increase in negative pressure to steady state. Both the foam dressing and the beaded dressing of the present disclosure show a lower actual pressure at the wound site than the pump set point as demonstrated in FIG. 5A, however, it is believed that the lower resistance to pressure presented by the wound dressing 102 of the present disclosure allows the negative wound pressure apparatus to draw a vacuum at the wound site faster than wound dressings using conventional material such as foams and gauzes. Eventually both dressings will achieve a steady steady-state pressure with the dressing with the least resistant to flow, in this case the present disclosure, reaching a higher actual vacuum level at the wound site than the more restrictive foam dressing.

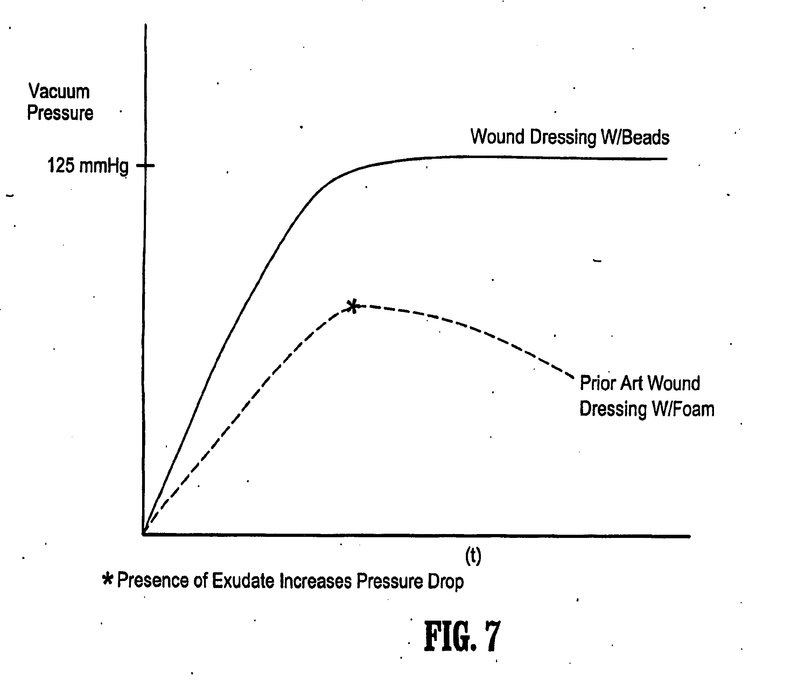

Resistance to fluid flow by the dressing can be further increased by the addition of fluid into the system. FIG. 7 shows vacuum level as measured at the wound site of a wet wound over time. Two lines are shown which represent the vacuum profiles of the wound dressing with beads 110 of the present disclosure and a prior art foam dressing. As can be seen, the dressing 102 with beads 110 of the present disclosure exhibits a faster draw down to vacuum pressure, shown by the steep slope of the line, and then reaches steady state and follows a generally horizontal path parallel to the set point of the vacuum pump. In contrast, the prior art wound dressing with foam has a slower increase in vacuum to a lower level of vacuum (similar to FIG. 6) and then a decrease as the foam becomes saturated with exudate from the wound. The saturated foam then has a higher pressure drop and may quickly exceed the capacity of the pump to maintain vacuum. The decrease in actual vacuum level at the wound site is due to the increased restriction to air flow caused by the wound fluid filling the cells of the foam. The beaded dressing 102 may also show a slight decrease in actual pressure at the wound, but the effect will be more prominent in the foam dressing because of the added effect of the collapsing cells. It is believed that this effect leads to a gradual increase in pressure (loss of vacuum at the wound site) which works contrary to the benefits of negative pressure wound therapy.

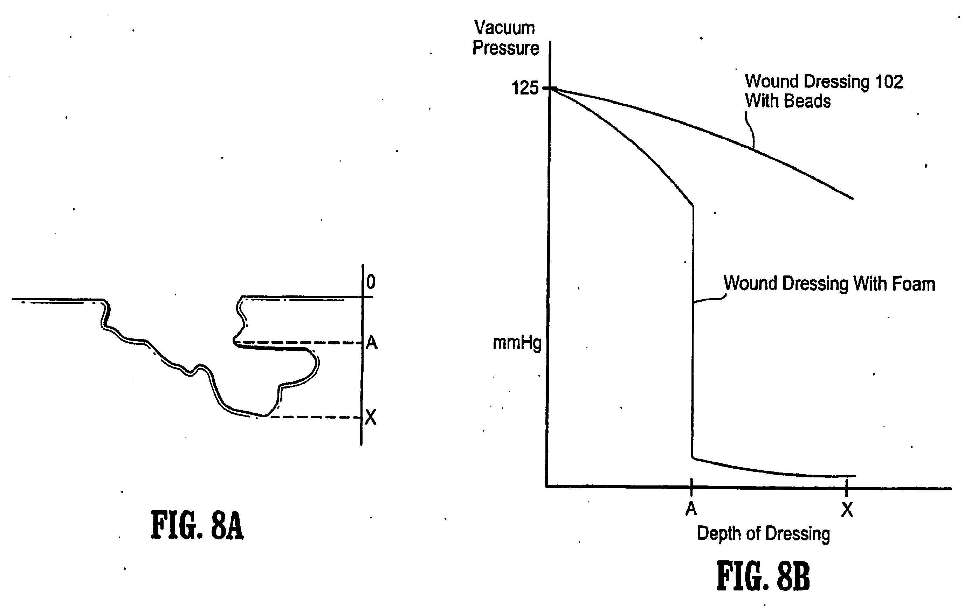

FIGS. 8A and 8B shows that in the case of a tunneling wound the drop off in vacuum level from the top surface of the dressing (0) to the bottom of the wound (x) (in cm) can be even more severe than as described in FIG. 5A and 5B. As negative pressure is applied to the wound, the narrowing part of the tunneling wound (a) may be pulled closed due to the vacuum pressure and the collapse a traditional foam dressing. If this is the case, the pressure profile will steadily decrease from the dressing surface to the pinch point and then drop off close to atmospheric pressure inside the tunneling portion of the wound. When the beaded dressing of the present disclosure is used, the beads 110 will not collapse under the negative pressure so a pathway to relieve pressure is maintained in the interstices and therapeutic levels of negative pressure are achieved throughout the entire wound including the tunnel.

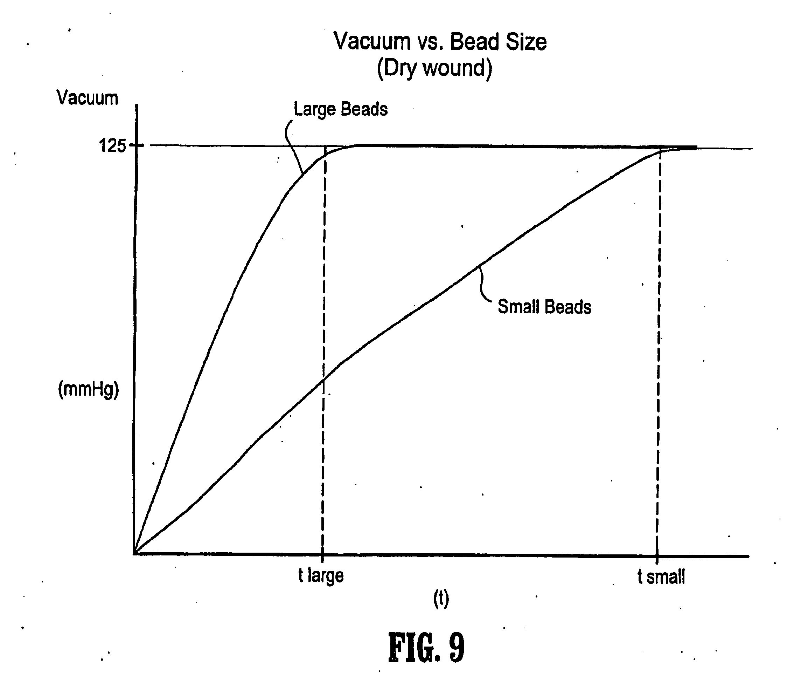

It is also important to note that the size of the beads 110 used in the dressing will affect the average pore size in the interstices, larger interstices for larger beads. The average pore size is directly related to the restriction to air or fluid flow through the dressing. FIG. 9 shows that if larger beads are used in the order of, e.g., 2mm, the pump down time will be shorter than if smaller beads (e.g. 0.5mm diameter) which will have smaller average pore sizes and more restriction to flow are used.

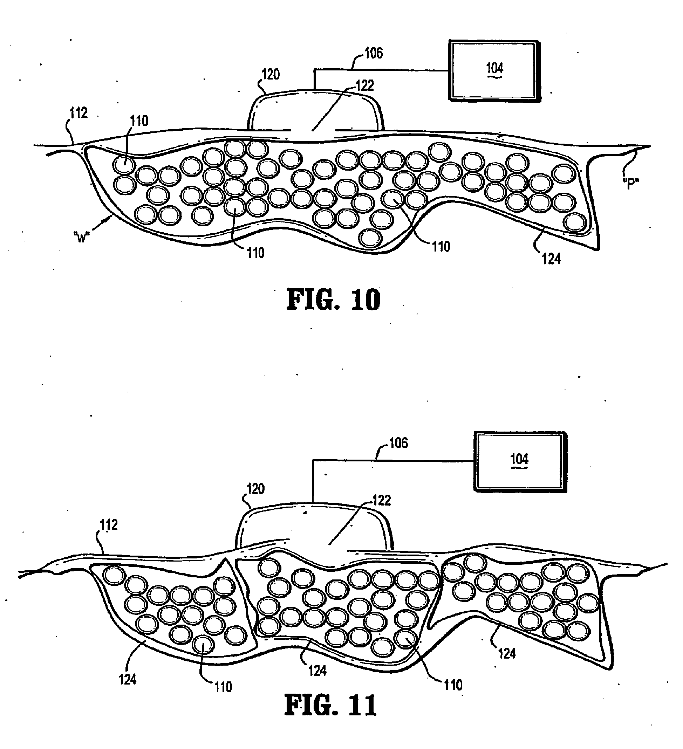

Referring now to FIG. 10, an alternate embodiment of the present disclosure is illustrated. In accordance with this embodiment, support member is in the form of an enclosure member or pouch 124 which houses beads 110. Pouch 124 may include an access opening to permit beads 110 to be positioned therein. Alternatively, pouch 124 may have an access door of the type aforedescribed in connection with outer member 112. In a further alternative, pouch 124 could be sealed with beads 110 prepackaged therein. Pouches 124 could be provided in a sheet or a roll to allow the wound dressing 100 to be sized to fit wounds of different shapes, depths, etc. The roll may have perforations demarking individual pouches which may be detached from the roll and sized accordingly. Pouch 124 facilitates handling of beads 110 by providing an enclosure in which the beads 110 are stored. Beads 110 are capable of movement within pouch 124 to readjust during healing and/or during cyclic application of the negative pressure.

FIG. 11 illustrates an alternate embodiment in which a number of enclosure pouches 124 are provided for receiving beads 110, and positionable within a wound bed "w". The increased number of pouches 124 may facilitate placement and removal of beads 110.

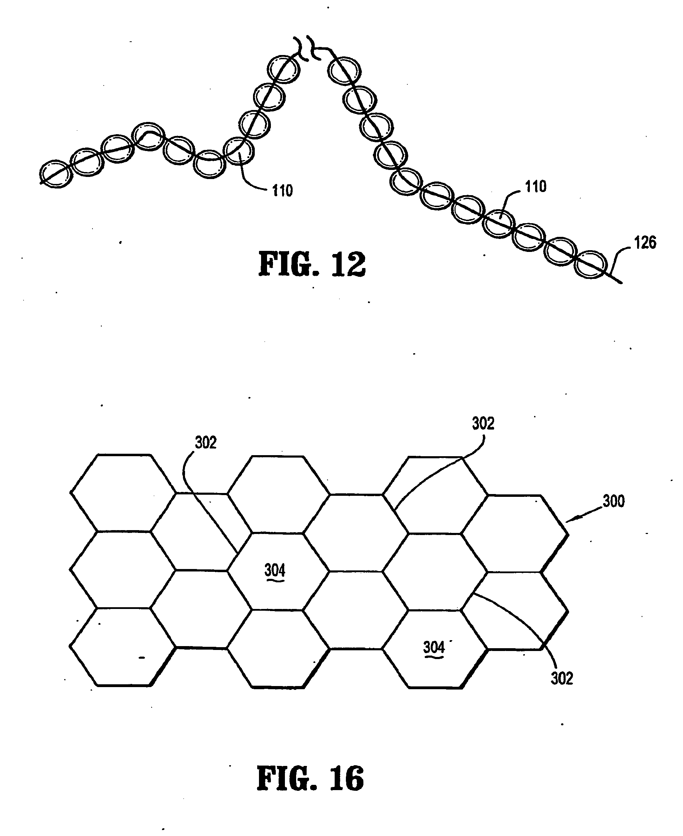

FIGS. 12 disclose an alternative embodiment of wound dressing of the present disclosure. In accordance with this embodiment, beads 110 are strung on an elongate member, suture or string 126, thus preventing the beads 110 from becoming loose prior to application in wound dressing 102 or loose when disposed in the wound bed "w" (FIG. 10). Elongate member 126 with beads 110 may be added to the wound bed "w" and positioned within support member 108. It is envisioned that elongate member 126 with beads 110 may be cut to fit a particular wound bed "w". Elongate member 126 may be formed of a bioabsorbable material, or alternatively, a nonabsorbable material. Suitable materials include a POLYSORB™ suture from U.S. Surgical, a divisional of Tyco Healthcare Group LP, synthetic polyester, catgut or non-absorbable including silk or nylon.

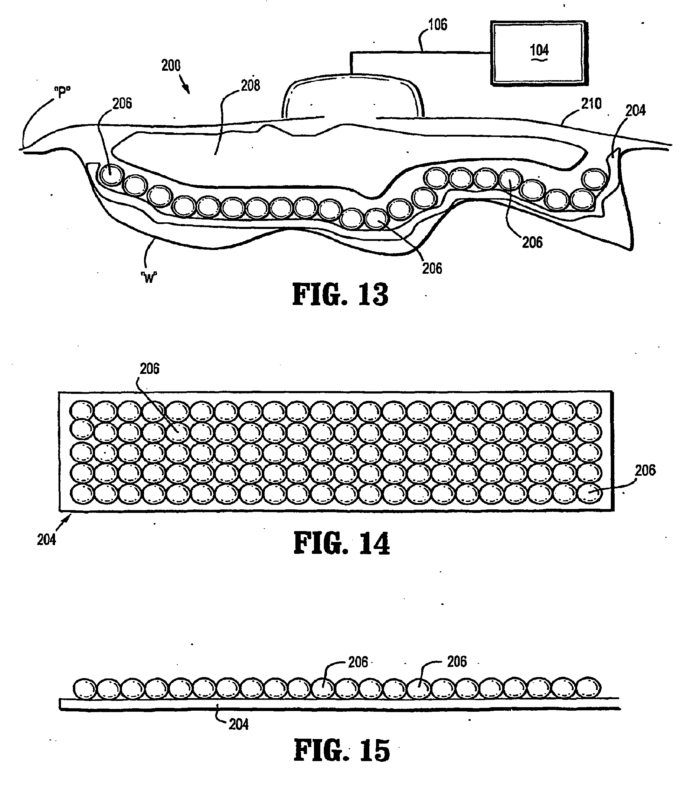

Referring now to FIGS. 13-15, another alternative embodiment of the wound dressing system of the present disclosure is illustrated. Wound dressing system 200 includes wound dressing 202 having support member in the form of a sheet 204, and beads 206 which are bonded to the sheet 204 (FIGS. 14 and 15). Sheet 204 is preferably formed from silicone although other materials are also envisioned. Beads 206 may be molded onto sheet 204 during manufacture or subsequently bonded onto sheet 204. Sheet 204 may be permeable to liquids, vapor, etc. or may have perforations to permit air and fluid to pass through from the wound bed "w" and into the conduit. Sheet 204 is substantially flexible and is configured to substantially conform to a wound bed "w" as shown in FIG. 13. Beads 206 and sheet 204 may contain anti-microbials or anti-infectives, such as silver or PHMB.

Wound dressing 202 may also include absorbent member 208. Absorbent member 208 may be fabricated from a material selected from the group consisting of foams, sponges, non-woven composite fabrics, cellulose fabrics, super absorbent polymers, and combinations thereof. Absorbent member 208 is intended to absorb and capture wound fluid and exudates. Exemplary materials used as the absorbent member 208 include the antimicrobial dressing sold under the trademark KERLIX™ by Kendall Corp., a division of TycoHealthcare. Those skilled in the art will recognize that the absorbent member 208 can be formed into any suitable shape. The only requirement as to shape is that absorbent member 208 is suitable to conform to a particular shape of the wound.

Wound dressing further includes outer member 210 which is substantially similar to outer member 112 discussed hereinabove in connection with the embodiment of FIG.1.

Referring now to FIG. 16, another alternative embodiment of the wound dressing system of the present disclosure is illustrated. In accordance with this embodiment, the support member and beads are replaced with lower or frame member 300. Frame member 300 is preferably a honeycomb structure defining a polygonal lattice support having a series of interconnected walls 302 with spaced open cells 304 extending therethrough. Frame member 300 is rigid and will not collapse under the force of negative pressure while spaced cells 304 permit the flow of fluid and air therethrough. Frame member 300 is preferably fabricated from silicon or another suitable biocompatible material. Frame member 300 is preferably molded into the particular lattice framework. Cells 304 may be hexagonal, square, triangular or even circular in configuration. As mentioned hereinabove, anti-microbials or anti-infectives, such as silver or PHMB could be disposed within cells 304 or incorporated into the surface of the framework of frame member 300.

## Claims

1. A wound dressing system (100, 200), which comprises:
a fluid permeable support member (108, 124, 204, 300), the support member configured for positioning within a wound ("w") and adapted to generally conform to a topography of the wound, and to permit exudates from the wound to pass therethrough;
a plurality of beads (110, 206) supported by the support member, the beads having sufficient rigidity to substantially maintain respective shapes thereof to thereby facilitate passage of the exudates through spaces defined between adjacent beads;
an outer member (112) adapted for positioning over the wound to substantially enclose the beads; and
a conduit (106) for supplying reduced pressure to the wound,
**characterized by** at least some of the plurality of beads each having a bore (118) therethrough, the bore configured to facilitate passage of the exudates or of air.

2. The wound dressing system (100, 200) according to claim 1 wherein the beads (110, 206) comprise at least one of glass and a polymeric material.

3. The wound dressing system (100, 200) according to claim 1 or 2 wherein at least some of the beads (110, 206) comprise an outer layer (116) of medicament.

4. The wound dressing system (100, 200) according to any preceding claim wherein the beads (110, 206) having the bores (118) each include a medicament disposed within the bore.

5. The wound dressing system (100, 200) according to any preceding claim wherein the beads (110, 206) are mounted to the support member (108, 124, 204, 300).

6. The wound dressing system (100, 200) according to claim 1 wherein the support member (108, 124, 204, 300) comprises a fabric material.

7. The wound dressing system (100, 200) according to claim 1 wherein the support member (108, 124, 204, 300) comprises a polymeric material.

8. The wound dressing system according to any preceding claim wherein the outer member (112) is adapted to form a substantially liquid-tight seal about the wound ("w").

9. The wound dressing system (100, 200) according to claim 8 wherein the outer member (112) comprises a semi-permeable material.

10. The wound dressing system (100, 200) according to any preceding claim further comprising a negative pressure source (104) in fluid communication with the conduit (106).

11. The wound dressing system according to Claim 10 wherein the negative pressure source includes a vacuum pump.

12. The wound dressing system (100, 200) according to any preceding claim wherein the support member (108, 124, 204, 300) is a pouch, the pouch housing the beads (110, 206).

13. The wound dressing system (100, 200) according to any preceding claim wherein at least some of the beads (110, 206) comprise at least one of antimicrobial agents, growth factors, medicaments, antibiotics, analgesics, and healing factors such as vitamins and nutrients.

14. The wound dressing system (100, 200) according to any preceding claim wherein at least some of the beads (110, 206) comprise a bioabsorbable polymer.

15. The wound dressing system (100, 200) according to claim 2 wherein the polymeric material is selected from the group consisting of polylactide (PLA), polyglycolide (PGA), Chitosan, polyethylene oxide (PEO) and polyethylene glycol (PEG).

16. The wound dressing system (100, 200) according to any preceding claim wherein at least some of the beads (110, 206) comprises a hydrogel coating.

17. The wound dressing system (100, 200) according to claim 1 wherein at least some of the beads (110, 206) comprises a hydrogel coating with a medicament.

18. The wound dressing system (100, 200) according to claim 17 wherein the medicament is selected from the group consisting of antimicrobial agents, growth factors, antibiotics, analgesics, and debridement agents.

## Patentansprüche

1. Ein Wundverbandsystem (100, 200), das Folgendes beinhaltet:
ein fluiddurchlässiges Stützelement (108, 124, 204, 300), wobei das Stützelement zur Positionierung innerhalb einer Wunde ("w") konfiguriert ist und angepasst ist, um sich im Allgemeinen einer Topographie der Wunde anzupassen, und um Exsudaten aus der Wunde zu ermöglichen, dort hindurch zu gehen;
eine Vielzahl von Perlen (110, 206), die durch das Stützelement gestützt werden, wobei die Perlen eine ausreichende Steifigkeit aufweisen, um im Wesentlichen ihre jeweiligen Formen beizubehalten, um dadurch den Durchgang der Exsudate durch zwischen angrenzenden Perlen definierte Räume zu erleichtern;
ein äußeres Element (112), das zur Positionierung über der Wunde angepasst ist, um die Perlen im Wesentlichen einzuschließen; und
einen Kanal (106), um reduzierten Druck an die Wunde zu liefern,
**dadurch gekennzeichnet, dass** mindestens einige der Vielzahl von Perlen jeweils eine Bohrung (118) dort hindurch aufweisen, wobei die Bohrung konfiguriert ist, um den Durchgang der Exsudate oder von Luft zu erleichtern.

2. Wundverbandsystem (100, 200) gemäß Anspruch 1, wobei die Perlen (110, 206) mindestens eines von Glas und einem Polymermaterial beinhalten.

3. Wundverbandsystem (100, 200) gemäß Anspruch 1 oder 2, wobei mindestens einige der Perlen (110, 206) eine äußere Schicht (116) Medikament beinhalten.

4. Wundverbandsystem (100, 200) gemäß einem der vorhergehenden Ansprüche, wobei die die Bohrungen (118) aufweisenden Perlen (110, 206) jeweils ein innerhalb der Bohrung angeordnetes Medikament umfassen.

5. Wundverbandsystem (100, 200) gemäß einem der vorhergehenden Ansprüche, wobei die Perlen (110, 206) an dem Stützelement (108, 124, 204, 300) angebracht sind.

6. Wundverbandsystem (100, 200) gemäß Anspruch 1, wobei das Stützelement (108, 124, 204, 300) ein Stoffmaterial beinhaltet.

7. Wundverbandsystem (100, 200) gemäß Anspruch 1, wobei das Stützelement (108, 124, 204, 300) ein polymeres Material beinhaltet.

8. Wundverbandsystem gemäß einem der vorhergehenden Ansprüche, wobei das äußere Element (112) angepasst ist, um eine im Wesentlichen flüssigkeitsdichte Dichtung um die Wunde ("w") zu bilden.

9. Wundverbandsystem (100, 200) gemäß Anspruch 8, wobei das äußere Element (112) ein halbdurchlässiges Material beinhaltet.

10. Wundverbandsystem (100, 200) gemäß einem der vorhergehenden Ansprüche, das ferner eine Unterdruckquelle (104) in Fluidkommunikation mit dem Kanal (106) beinhaltet.

11. Wundverbandsystem gemäß Anspruch 10, wobei die Unterdruckquelle eine Vakuumpumpe umfasst.

12. Wundverbandsystem (100, 200) gemäß einem der vorhergehenden Ansprüche, wobei das Stützelement (108, 124, 204, 300) ein Beutel ist, wobei der Beutel die Perlen (110, 206) unterbringt.

13. Wundverbandsystem (100, 200) gemäß einem der vorhergehenden Ansprüche, wobei mindestens einige der Perlen (110, 206) mindestens eines der Folgenden beinhalten:
antimikrobielle Wirkstoffe, Wachstumsfaktoren, Medikamente, Antibiotika, Analgetika und Heilungsfaktoren wie etwa Vitamine und Nährstoffe.

14. Wundverbandsystem (100, 200) gemäß einem der vorhergehenden Ansprüche, wobei mindestens einige der Perlen (110, 206) ein bioabsorbierbares Polymer beinhalten.

15. Wundverbandsystem (100, 200) gemäß Anspruch 2, wobei das polymere Material aus der Gruppe, bestehend aus Polylactid (PLA), Polyglycolid (PGA), Chitosan, Polyethylenoxid (PEO) und Polyethylenglycol (PEG), ausgewählt ist.

16. Wundverbandsystem (100, 200) gemäß einem der vorhergehenden Ansprüche, wobei mindestens einige der Perlen (110, 206) eine Hydrogelbeschichtung beinhalten.

17. Wundverbandsystem (100, 200) gemäß Anspruch 1, wobei mindestens einige der Perlen (110, 206) eine Hydrogelbeschichtung mit einem Medikament beinhalten.

18. Wundverbandsystem (100, 200) gemäß Anspruch 17, wobei das Medikament aus der Gruppe, bestehend aus antimikrobiellen Wirkstoffen, Wachstumsfaktoren, Antibiotika, Analgetika und Wundreinigungswirkstoffen, ausgewählt ist.

## Revendications

1. Un système de pansement pour plaie (100, 200), qui comprend :
un élément de support perméable aux fluides (108, 124, 204, 300), l'élément de support étant configuré pour être placé au sein d'une plaie (« w ») et conçu pour épouser généralement une topographie de la plaie, et pour laisser passer des exsudats émanant de la plaie à travers lui ;
une pluralité de billes (110, 206) soutenues par l'élément de support, les billes étant suffisamment rigides pour garder substantiellement leurs configurations respectives afin de faciliter de ce fait le passage des exsudats dans des espaces définis entre des billes adjacentes ;
un élément externe (112) conçu pour être placé par-dessus la plaie pour renfermer substantiellement les billes ; et
un conduit (106) destiné à apporter une pression réduite dans la plaie,
**caractérisé en ce qu'**au moins certaines billes de la pluralité de billes présentent chacune un alésage (118) à travers elles, l'alésage étant configuré pour faciliter le passage des exsudats ou d'air.

2. Le système de pansement pour plaie (100, 200) selon la revendication 1 dans lequel les billes (110, 206) comprennent au moins un matériau parmi du verre et un matériau polymère.

3. Le système de pansement pour plaie (100, 200) selon la revendication 1 ou la revendication 2 dans lequel au moins certaines des billes (110, 206) comprennent une couche externe (116) de médicament.

4. Le système de pansement pour plaie (100, 200) selon n'importe quelle revendication précédente dans lequel les billes (110, 206) présentant les alésages (118) comportent chacune un médicament disposé au sein de l'alésage.

5. Le système de pansement pour plaie (100, 200) selon n'importe quelle revendication précédente dans lequel les billes (110, 206) sont montées sur l'élément de support (108, 124, 204, 300).

6. Le système de pansement pour plaie (100, 200) selon la revendication 1 dans lequel l'élément de support (108, 124, 204, 300) comprend un matériau en tissu.

7. Le système de pansement pour plaie (100, 200) selon la revendication 1 dans lequel l'élément de support (108, 124, 204, 300) comprend un matériau polymère.

8. Le système de pansement pour plaie selon n'importe quelle revendication précédente dans lequel l'élément externe (112) est conçu pour former un joint substantiellement imperméable aux liquides autour de la plaie (« w »).

9. Le système de pansement pour plaie (100, 200) selon la revendication 8 dans lequel l'élément externe (112) comprend un matériau semi-perméable.

10. Le système de pansement pour plaie (100, 200) selon n'importe quelle revendication précédente comprenant en sus une source de pression négative (104) en communication fluidique avec le conduit (106).

11. Le système de pansement pour plaie selon la revendication 10 dans lequel la source de pression négative comporte une pompe à vide.

12. Le système de pansement pour plaie (100, 200) selon n'importe quelle revendication précédente dans lequel l'élément de support (108, 124, 204, 300) est une poche, la poche abritant les billes (110, 206).

13. Le système de pansement pour plaie (100, 200) selon n'importe quelle revendication précédente dans lequel au moins certaines des billes (110, 206) comprennent au moins un élément parmi des agents antimicrobiens, des facteurs de croissance, des médicaments, des antibiotiques, des analgésiques, et des facteurs de cicatrisation tels que des vitamines et des nutriments.

14. Le système de pansement pour plaie (100, 200) selon n'importe quelle revendication précédente dans lequel au moins certaines des billes (110, 206) comprennent un polymère bioabsorbable.

15. Le système de pansement pour plaie (100, 200) selon la revendication 2 dans lequel le matériau polymère est sélectionné dans le groupe consistant en polylactide (PLA), polyglycolide (PGA), Chitosane, oxyde de polyéthylène (PEO) et polyéthylèneglycol (PEG).

16. Le système de pansement pour plaie (100, 200) selon n'importe quelle revendication précédente dans lequel au moins certaines des billes (110, 206) comprennent un revêtement en hydrogel.

17. Le système de pansement pour plaie (100, 200) selon la revendication 1 dans lequel au moins certaines des billes (110, 206) comprennent un revêtement en hydrogel avec un médicament.

18. Le système de pansement pour plaie (100, 200) selon la revendication 17 dans lequel le médicament est sélectionné dans le groupe consistant en agents antimicrobiens, facteurs de croissance, antibiotiques, analgésiques, et agents de débridement.
